# EUROPEAN PATENT APPLICATION

(11) **EP 4 793 642 A1**
(43) Date of publication of application: **19.08.2026**
(21) Application number: 24877509.0
(22) Date of filing: 08.10.2024
(51) Int. Cl.: G01N 33/50, C12N 5/071

(54) **DAMAGED SKIN ORGANOID MODEL AND DRUG SCREENING METHOD USING SAME**

(30) Priority: 10.10.2023 KR 20230134662
(71) Applicant: Kangstem Biotech Co., Ltd., Seoul 06179 (KR)
(72) Inventor: KANG, Kyung Sun, Seoul 06338 (KR); KIM, Minji, Seoul 04026 (KR)
(74) Representative: Yip, Matthew Wing Yu
(86) International application number: PCT/KR2024/015315
(87) International publication number: WO 2025/079973

(57) **Abstract**

The present invention relates to a skin damage organoid model and a drug screening method using the same, which utilize a technology of producing an air-liquid interface skin organoid forming skin cells, appendages, nerve cells, and fat cells in a structure similar to actual skin, and creates damaged skin by irradiating the skin organoid with UV rays that mimic UV rays reaching the actual Earth surface. In the present invention, since the skin damage organoid model is irradiated with solar light-mimicking UV rays, including 94.5% UV-A and 5.5% UV-B, the skin barrier is damaged and the activity of a support protein degradation enzyme is increased, degradation of collagen is promoted, and the secretion of pro-inflammatory cytokines is increased, which mimic the characteristics of skin damaged by UV rays well, this model can be effectively used as a model of UV-induced damage and aging, and is expected to be a useful technology for screening potential therapeutic drugs.

## Description

### Technical Field

This application claims priority to and the benefit of Korean Patent Application No. 10-2023-0134662, filed on October 10, 2023, the disclosure of which is incorporated herein by reference in its entirety.

The present invention relates to a skin damage organoid model and drug screening methods using the same, a method of manufacturing a photoaging-like skin model due to UV-induced damage by irradiating a differentiated human skin organoid with UV rays that mimic the UV rays that actually reach the Earth surface, and a method of evaluating the potential efficiency of a therapeutic drug.

The present invention was completed under Project No. 00215812 (1711195569) with the support of the Ministry of Science and ICT of the Republic of Korea.

### Background Art

With the recent improvement in living standards, modern people are not only concerned with maintaining the health of their bodies, but also with maintaining healthy skin. Therefore, there is a lot of interest in not only skin beauty, but also skin damage and aging. The skin is the largest organ in the human body, accounting for about 15% of the body weight, covering the outer surface, and direct contacting the outside. Skin cells are damaged by many external elements that try to enter the body. Among them, UV exposure is a well-known extrinsic aging factor in the skin, and it is known that UV exposure alone can cause a great deal of damage to the skin. Recently, the amount of UV rays reaching the Earth surface is increasing due to the depletion of the ozone layer which plays a protective role at the top of the atmosphere, and this increase can potentially cause skin damage.

Currently, methods for improving phenomena relating to UV-induced damage and aging are being actively studied, and particularly, efforts are being made to discover key mechanisms that cause skin damage or aging and identify materials that mitigate this phenomenon.

### [Related Art Documents]

### [Patent Documents]

Korean Unexamined Patent Application Publication No. 10-2023-0115654 (published on August 03, 2023)

### Disclosure

### Technical Problem

The present invention is directed to providing a method of preparing a skin damage or hair follicle damage organoid through solar light-mimicking UV irradiation.

The present invention is also directed to providing a skin damage organoid or a hair follicle damage organoid, which is prepared by the above preparation method.

The present invention is also directed to providing a method of screening a skin damage treatment agent, preventive agent or improvement agent using the skin damage organoid.

The present invention is also directed to providing a method of screening a hair growth promoter, a hair loss preventive agent, a hair loss relief agent, or a hair loss treatment agent using the hair follicle damage organoid.

The present invention is also directed to providing a biomarker composition for detecting hair follicle damage, including NF-kB as an active ingredient.

The present invention is also directed to providing a kit for detecting hair follicle damage, including an agent that can detect NF-kB.

The present invention is also directed to providing a method of providing information required for detection of hair follicle damage by measuring an expression level of NF-kB.

The present invention is also directed to providing a pharmaceutical composition for a hair growth promoter, a hair loss preventive agent, a hair loss relief agent, or a hair loss treatment agent, including an NF-kB activation or expression inhibitor as an active ingredient.

The present invention is also directed to providing a pharmaceutical composition or cosmetic composition for a hair removal agent, including an NF-kB activation or expression promoter as an active ingredient.

The present invention is also directed to providing a method of promoting hair growth, preventing hair loss, reducing hair loss, or treating hair loss, which includes administering an effective amount of an NF-kB activation or expression inhibitor to a subject in need thereof.

The present invention is also directed to providing a method of removing hair, which includes administering an effective amount of an NF-kB activation or expression promoter to a subject in need thereof.

### Technical Solution

To achieve the above objects, the present invention provides a method of preparing a skin damage organoid, which includes the following steps: (1) culturing a pluripotent stem cell-derived organoid in the presence of a Wnt agonist; (2) culturing the cultured product obtained in (1) in a medium for skin organoid maturation; (3) preparing a skin organoid by cutting the cultured product obtained in (2) and culturing it at an air-liquid interface; and (4) irradiating the skin organoid with solar light-mimicking UV rays, wherein the Wnt agonist is added at the beginning of the induction of differentiation of non-neural ectoderm into cranial neural crest cells (CNCCs).

In addition, the present invention provides a skin damage organoid or a hair follicle damage organoid, which is prepared by the preparation method.

In addition, the present invention provides a method of screening a skin damage treatment agent, preventive agent or improvement agent, which includes treating the skin damage organoid with a candidate material for a skin damage treatment agent, preventive agent or improvement agent.

In addition, the present invention provides a method of screening a hair growth promoter, a hair loss preventive agent, a hair loss relief agent, or a hair loss treatment agent, which includes treating the hair follicle damage organoid with a candidate material for a hair growth promoter, a hair loss preventive agent, a hair loss relief agent or a hair loss treatment agent.

In addition, the present invention provides a biomarker composition for detecting hair follicle damage, including NF-kB as an active ingredient.

In addition, the present invention provides a kit for detecting hair follicle damage, including an agent that can detect NF-kB.

In addition, the present invention provides a method of providing information required for detection of hair follicle damage, which includes: (1) measuring the expression level of NF-kB from an isolated sample; (2) comparing the expression level of NF-kB with a control sample; and (3) determining that hair follicles are damaged when the expression level of NF-kB is higher than that of the control sample.

In addition, the present invention provides a pharmaceutical composition for a hair growth promoter, a hair loss preventive agent, a hair loss relief agent, or a hair loss treatment agent, which includes a NF-kB activation or expression inhibitor as an active ingredient.

In addition, the present invention provides a pharmaceutical composition for a hair removal agent, which includes a NF-kB activation or expression promoter as an active ingredient.

In addition, the present invention provides a cosmetic composition for a hair removal agent, which includes a NF-kB activation or expression promoter as an active ingredient.

In addition, the present invention provides a method of promoting hair growth, preventing hair loss, reducing hair loss, or treating hair loss, which includes administering an effective amount of an NF-kB activation or expression inhibitor to a subject in need thereof.

In addition, the present invention provides a method of removing hair, which includes administering an effective amount of an NF-kB activation or expression promoter to a subject in need thereof.

The present invention provides a method of preparing a skin damage organoid, which includes the following steps: (1) culturing a pluripotent stem cell-derived organoid in the presence of a Wnt agonist; (2) culturing the cultured product obtained in (1) in a medium for skin organoid maturation; (3) preparing a skin organoid by cutting the cultured product obtained in (2) and culturing it at an air-liquid interface; and (4) irradiating the skin organoid with solar light-mimicking UV rays, wherein the Wnt agonist is added at the beginning of the induction of differentiation of non-neural ectoderm into CNCCs.

The skin damage organoid may be a hair follicle damage organoid with damaged hair follicles among the skin appendages, but the present invention is not limited thereto.

The solar light-mimicking UV rays may include 50 to 94.5% of UV-A and 5.5 to 50% of UV-B, but the present invention is not limited thereto.

The solar light-mimicking UV ray may be treated at an sUV dose of 25 to 75 kJ/m² after culturing the skin organoid under a dry condition to mature the stratum corneum, but the present invention is not limited thereto.

The Wnt agonist may be selected from the group consisting of CHIR-99021, Wnt3A, WNT5A, and RSPO1, but the present invention is not limited thereto.

The Wnt agonist may be added on day 5 to day 7 of the culture of pluripotent stem cells, but the present invention is not limited thereto.

The medium for skin organoid maturation may include one or more ingredients selected from the group consisting of GlutaMAX, 2-mercaptoethanol, B-27, N2, and Normosin, but the present invention is not limited thereto.

In (3), the cultured product obtained in (2) may cut into four equal-sized pieces and cultured at the air-liquid interface on a collagen-coated Transwell culture insert such that the dermis faces the collagen side and the epidermis is exposed to the air, and preferably, the air-liquid interface culture is performed in a medium for skin organoid maturation, but the present invention is not limited thereto.

The skin damage organoid or hair follicle damage organoid may have skin or hair follicles damaged by NF-kB activation, but the present invention is not limited thereto.

In the skin damage organoid, a protein forming the epidermal barrier selected from the group consisting of filaggrin, loricrin, and CK10 may be down-regulated, but the present invention is not limited thereto.

In the skin damage organoid, decreased expression of type I collagen and increased expression of MMP-1 in the dermis may occur, but the present invention is not limited thereto.

The hair follicle damage organoid may have decreased expression of one or more genes selected from the group consisting of a-SMA, which is a dermal sheath marker, KRT5 or KRT15, which is an outer root sheath marker, LHX2, which is a hair follicle stem cell marker, and SOX2, which is a dermal papilla marker, but the present invention is not limited thereto.

The hair follicle damage organoid may have increased gene expression of a pro-inflammatory cytokine, such as COX-2, TNF-α, or IL-1β, but the present invention is not limited thereto.

"Air-liquid interface (ALI) culture" used herein may be a culture in a partially open culture vessel or a culture vessel partially filled with a medium, but the present invention is not limited thereto. The air-liquid interface culture may expose the surface of cells or organoids to the air.

"Filaggrin" used herein is one of several structural proteins expressed by keratinocytes during the differentiation stage and is involved in differentiation from the stratum basale to the stratum corneum of the epidermis. Filaggrin is a major component of a natural moisturizing factor (NMF) that is essential for maintaining moisture in skin tissue, and is used as a key indicator of skin moisture retention and skin barrier function.

"Loricrin" used herein is one of the skin barrier proteins that are expressed by keratinocytes in the differentiation stage.

In addition, the present invention provides a skin damage organoid prepared by the preparation method.

In addition, the present invention provides a method of screening a skin damage treatment agent, preventive agent or improvement agent, which includes treating the skin damage organoid with a candidate material for a skin damage treatment agent, preventive agent or improvement agent.

In addition, the present invention provides a hair follicle damage organoid prepared by the preparation method.

In the hair follicle damage organoid, the expression of one or more genes selected from the group consisting of a-SMA, which is a dermal sheath marker, KRT5 or KRT15, which is an outer root sheath marker, LHX2, which is a hair follicle stem cell marker, and SOX2, which is a dermal papilla marker, may be reduced, but the present invention is not limited thereto.

In addition, the present invention provides a method of screening a hair growth promoter, a hair loss preventive agent, a hair loss relief agent, or a hair loss treatment agent, which includes treating the hair follicle damage organoid with a candidate material for a hair growth promoter, a hair loss preventive agent, a hair loss relief agent, or a hair loss treatment agent.

The candidate may inhibit NF-kB activation in the hair follicle damage organoid, but the present invention is not limited thereto.

The term "candidate material" used in the screening method of the present invention refers to an unknown candidate material used in screening to examine whether it affects the expression level of a gene or the expression or activity of a protein. The sample includes a chemical, a nucleotide, antisense RNA, small interference RNA (siRNA), and an extract of a natural substance, but the present invention is not limited thereto.

The term "organoid" used herein refers to a three-dimensional aggregate of one or more cell types that mimics the surface appearance or actual structure or function of tissue or an organ. In addition, an organoid can similarly reproduce the physiological function of the human body, and by constructing an organoid from a patient's tissue, disease modeling based on a patient's genetic information, drug screening through repeated testing, etc. can be performed.

In the present invention, "organoid culture" includes all actions that can generate or maintain organoids. For example, organoid culture may mean differentiating cells or cells isolated from specific tissue into tissue or organ cells with a specific function, and/or causing the organoids to survive, grow, or proliferate.

In addition, the present invention provides a biomarker composition for detecting hair follicle damage, including NF-kB as an active ingredient.

In addition, the present invention provides a kit for detecting hair follicle damage, including an agent that can detect NF-kB.

The agent may be one or more selected from the group consisting of a primer, a probe, an antisense oligonucleotide, an aptamer, and an antibody, which are specific for NF-kB, but the present invention is not limited thereto.

The term "primer" used herein refers to a nucleic acid sequence with a short free 3'-hydroxyl group, and a short nucleic acid sequence that is able to form a base pair with a complementary template and acts as a starting point for template strand replication. A primer can initiate DNA synthesis in the presence of a reagent for polymerization (i.e., a DNA polymerase or reverse transcriptase) and four different nucleoside triphosphates in an appropriate buffer at an appropriate temperature. PCR conditions, and the lengths of sense and antisense primers may be appropriately selected according to techniques known in the art.

The term "probe" used herein refers to a nucleic acid fragment, such as RNA or DNA, which is several to several hundred bases long, specifically binds to mRNA, and is labeled to confirm the presence and expression level of specific mRNA. Probes may be produced in the form of an oligonucleotide probe, a single-stranded DNA probe, a double-stranded DNA probe, or an RNA probe. Selection and hybridization conditions for proper probes may be appropriately selected according to the techniques known in the art.

The term "antisense oligonucleotide" used herein refers to an oligomer having a sequence of nucleotide bases and a backbone between subunits, which typically allows the formation of mRNA and RNA : oligomer heteroduplexes in a target sequence by hybridizing an antisense oligomer with the target sequence in RNA through Watson-Crick base pairing. An oligomer may have exact sequence complementarity or approximate complementarity to the target sequence. There antisense oligomers may block or inhibit the translation of mRNA and change the processing of mRNA for producing splice variants of mRNA.

The term "aptamer" used herein refers to a type of polynucleotide composed of a special type of single-stranded nucleic acid (DNA, RNA or a modified nucleic acid) having a stable tertiary structure in itself and the characteristic of binding to a target molecule with high affinity and specificity. As described above, an aptamer may replace an antibody because it is composed of a polynucleotide, which specifically binds to an antigenic material, like an antibody, has higher stability than a protein, has a simple structure, and is easily synthesized.

The term "antibody" used herein, as a term known in the art, refers to a specific immunoglobulin which is directed against an antigenic site. The antibody used in the present invention means an antibody specifically binding to a biomarker, and may be prepared by a conventional method known in the art. The antibody may be a polyclonal or monoclonal antibody, and includes all immunoglobulin antibodies. The antibody refers to a complete form with two full-length light chains and two full-length heavy chains. In addition, the antibody also includes a special antibody such as a humanized antibody.

In addition, the present invention provides a method of providing information required for detection of hair follicle damage, which includes (1) measuring an expression level of NF-kB from an isolated sample; (2) comparing the expression level of NF-kB with that of a control sample; and (3) determining that hair follicles are damaged when the expression level of NF-kB is higher than that of the control sample.

The term "isolated sample" used herein includes a sample such as tissue, cells, whole blood, serum, plasma, saliva, sputum, cerebrospinal fluid, or urine that differs from the control in terms of the expression level of NF-kB, which is a biomarker of the present invention, but the present invention is not limited thereto.

In addition, the present invention provides a pharmaceutical composition for a hair growth promoter, a hair loss preventive agent, a hair loss relief agent, or a hair loss treatment agent, which includes an NF-kB activation or expression inhibitor as an active ingredient.

The NF-kB activation inhibitor may be selected from the group consisting of a small molecule compound, a peptide, a peptide mimetic, an aptamer, an antibody, and a natural substance, which specifically bind to the NF-kB protein, and the NF-kB expression inhibitor may be selected from the group consisting of an antisense nucleotide, small interfering RNA (siRNA), and short hairpin RNA (shRNA) which complementarily bind to mRNA of the NF-kB gene, but the present invention is not limited thereto.

In addition, the present invention provides a pharmaceutical composition for a hair removal agent, which includes an NF-kB activation or expression promoter as an active ingredient.

The NF-kB activation promoter may be selected from the group consisting of a small molecule compound, a peptide, a peptide mimetic, an aptamer, an antibody, and a natural substance, which specifically bind to the NF-kB protein, and the NF-kB expression promoter may be selected from the group consisting of an adeno-associated virus (AAV), adenovirus, lentivirus, and retrovirus, which include an NF-kB coding gene increasing the expression of mRNA of the NF-kB gene.

The pharmaceutical composition of the present invention may include a chemical, a nucleotide, an antisense, an siRNA oligonucleotide, and a natural substance extract as an active ingredient. The pharmaceutical composition or combined preparation of the present invention may be prepared using a pharmaceutically suitable and physiologically acceptable adjuvant, in addition to the active ingredient, and as the adjuvant, a solubilizing agent such as an excipient, a disintegrant, a sweetener, a binder, a coating agent, a bulking agent, a lubricant, or a flavoring agent may be used. The pharmaceutical composition of the present invention may be prepared by further including one or more pharmaceutically acceptable carriers in addition to the active ingredient for administration. As the pharmaceutically acceptable carrier used in a composition prepared as a liquid, saline, sterile water, Ringer's solution, buffered saline, an albumin injection solution, a dextrose solution, a maltodextrin solution, glycerol, ethanol, and a mixture of one or more thereof, which are suitable for sterilization and the living body, may be used, and if needed, other conventional additives such as antioxidant, a buffer, a bacteriostat, etc. may be added. In addition, the composition of the present invention may be formulated into an injectable formulation such as an aqueous solution, a suspension, or an emulsion, a pill, a capsule, a granule, or a tablet by additionally adding a diluent, a dispersant, a surfactant, a binder, and a lubricant.

Dosage forms of the pharmaceutical composition of the present invention may include granules, powder, coated tablets, tablets, capsules, suppositories, a syrup, a juice, a suspension, an emulsion, a drop, an injectable liquid, and a sustained release formulation of an active compound. The pharmaceutical composition of the present invention may be administered via conventional ways, for example, intravenous, intraperitoneal, intramuscular, intraarterial, intrasternal, percutaneous, intranasal, inhalation, topical, rectal, oral, intraocular, and intradermal routes. The effective amount of the active ingredient of the present invention refers to an amount required for the prevention or treatment of a disease. Accordingly, the effective amount of the active ingredient of the pharmaceutical composition of the present invention may be adjusted according to various factors, including the type of disease, the severity of the disease, the contents of an active ingredient and other ingredients contained in a composition, the type of formulation, a patient's age, body weight, general health condition, sex, and diet, the time of administration, an administration route, the secretion rate of a composition, the duration of treatment, and a concurrent drug. Although not limited thereto, for adults, when administered once a day or several times a day, the composition of the present invention may be administered at 0.1 ng/kg to 10 g/kg in the case of a compound, 0.1 ng/kg to 10 g/kg in the case of a polypeptide, protein, or antibody, or 0.01 ng/kg to 10 g/kg in the case of an antisense nucleotide, siRNA, shRNAi, or miRNA.

In addition, the present invention provides a cosmetic composition for a hair removal agent, which includes a NF-kB activation or expression promoter as an active ingredient.

The cosmetic composition of the present invention may include conventional adjuvants such as a stabilizer, a solubilizer, a vitamin, a pigment, and a fragment, and carriers, in addition to the active ingredient.

Dosage forms of the cosmetic composition may be any one that can be produced conventionally in the art, and may be selected from the group consisting of a topical ointment, a cream, an emollient, a nourishing toner, a pack, an essence, a hair tonic, a shampoo, a rinse, a hair conditioner, a hair treatment, a gel, a skin lotion, a skin softener, a skin toner, an astringent, a lotion, a milk lotion, a moisture lotion, a nourishing lotion, a massage cream, a nourishing cream, an eye cream, a moisturizing cream, a hand cream, a foundation, a nourishing essence, a sunscreen, soap, a cleansing foam, a cleansing lotion, a cleansing cream, a body lotion, and a body cleanser, but the present invention is not limited thereto. The composition of each dosage form may contain various bases and additives, which are suitable and required for preparation of the dosage form, and the types and amounts of these components may be easily selected by those of ordinary skill in the art.

When the dosage form is a paste, a cream, or a gel, as a carrier component, an animal oil, a vegetable oil, wax, paraffin, starch, tragacanth, a cellulose derivative, polyethylene glycol, silicone, bentonite, silica, talc, or zinc oxide may be used.

When the dosage form is a powder or spray, as a carrier component, lactose, talc, silica, aluminum hydroxide, calcium silicate or polyamide powder may be used, and particularly, for a spray, a propellant such as chlorofluorohydrocarbon, propane/butane, or dimethyl ether may be further included.

When the dosage form is a solution or an emulsion, as a carrier component, a solvent, a solubilizer or an emulsifier may be used, and for example, water, ethanol, isopropanol, ethyl carbonate, ethyl acetate, benzyl alcohol, benzyl benzoate, propylene glycol, 1,3-butylglycol oil, a glycerol aliphatic ester, polyethylene glycol, or a fatty acid ester of sorbitan may be used.

When the dosage form is a suspension, as a carrier component, a liquid diluent such as water, ethanol, or propylene glycol, a suspending agent such as ethoxylate isostearyl alcohol, polyoxyethylene sorbitol ester, and polyoxyethylene sorbitan ester, microcrystalline cellulose, aluminum metahydroxide, bentonite, agar or tragacanth may be used.

In addition, the present invention provides a method of promoting hair growth, preventing hair loss, reducing hair loss, or treating hair loss, which includes administering an effective amount of an NF-kB activation or expression inhibitor to a subject in need thereof.

The NF-kB activation inhibitor may be selected from the group consisting of a small molecule compound, a peptide, a peptide mimetic, an aptamer, an antibody, and a natural substance, which specifically bind to the NF-kB protein, and the NF-kB expression inhibitor may be selected from the group consisting of an antisense nucleotide, small interfering RNA (siRNA), and short hairpin RNA (shRNA) which complementarily bind to mRNA of the NF-kB gene, but the present invention is not limited thereto.

In addition, the present invention provides a hair removal method, which includes administering the effective amount of a NF-kB activation or expression promoter to a subject in need thereof.

The NF-kB activation promoter may be selected from the group consisting of a small molecule compound, a peptide, a peptide mimetic, an aptamer, an antibody, and a natural substance, which specifically bind to the NF-kB protein, and the NF-kB expression promoter may be selected from the group consisting of adeno-associated virus (AAV), adenovirus, lentivirus, and retrovirus, which include an NF-kB coding gene increasing the expression of mRNA of the NF-kB gene.

### Advantageous Effects

The present invention relates to a skin damage organoid model and a drug screening method using the same, and therefore, skin damaged by the external environment (UV rays) was modeled by utilizing a human induced pluripotent stem cell-derived skin organoid. By utilizing organoid technology that mimics the actual human development process, a skin organoid that contains cells constituting skin, cells constituting hair follicles, and all microstructures without modification was produced and irradiated with UV rays to mimic an external stimulus in an actual external environment, establishing a technology for modeling damaged skin in vitro. Therefore, through this technology, the mechanism causing damage and aging caused by UV rays can be confirmed in vitro, and a platform that can derive a candidate material capable of alleviating this was developed. The platform established through the present invention can be applied as a platform that can evaluate and derive the mechanism of disease occurrence and the efficacy of various drug candidates in the future.

### Description of Drawings

FIG. 1 illustrates a skin organoid differentiation method using a human induced pluripotent stem cell line.
FIG. 2 shows the results of comparing the skin structure shown in a fabricated skin organoid and the skin structure of a human adult.
FIG. 3A shows the results of investigating the effect of various sUV doses on SkOs.
FIG. 3B shows the results of confirming the effect of sUV exposure on the epidermis, the dermis, or hair follicles of SkOs.
FIG. 3C shows the results of confirming that the sUV effect extends to the deep dermal layer of SkOs.
FIG. 4A shows the results of investigating the effect of sUV exposure on skin appendages in the dermis of SkOs.
FIG. 4B shows the results of investigating whether sUV exposure affects hair follicles and induces an inflammatory response in the dermis of SkOs.
FIG. 5A shows the results of investigating the effect of UCB-Exos on photodamage using a sUV-exposed SkO model.
FIG. 5B shows the results of confirming that epidermal damage caused by sUV exposure was improved in SkOs by effectively reducing the epidermal thickness and alleviating the disruption of skin barrier proteins (filaggrin and loricrin) due to UCB-Exos treatment.
FIG. 6 shows the results of investigating the effect of UCB-Exos on hair follicles in SkOs.
FIG. 7A shows the results of confirming that sUV rays induce the phosphorylation of IκBα and then IκBα degradation in the cytoplasmic fraction of SkOs.
FIG. 7B shows the results of confirming that sUV rays induce IκBα phosphorylation and then IκBα degradation in the cytoplasmic fraction of SkOs.
FIG. 7C shows the results of confirming that UCB-Exos inhibits IκB degradation and NF-κB activation, induced by sUV exposure, to alleviate an inflammatory response in fibroblasts and keratinocytes present in hair follicles.
FIG. 8A shows the results of confirming that the inhibition of NF-κB activation by UCB-Exos has the potential to alleviate the detrimental effects of SASP, thereby creating a more favorable environment for tissue regeneration.
FIG. 8B shows the results of confirming that the inhibition of NF-κB activation by UCB-Exos has the potential to alleviate the detrimental effects of SASP, thereby creating a more favorable environment for tissue regeneration.
FIG. 8C shows the results of confirming that UCB-Exos effectively recovered hair follicles damaged by sUV rays through NF-κB inhibition.
FIG. 9 shows the results of confirming the hair follicle growth promoting effect by an NF-κB signaling inhibitor in a normal hair follicle organoid.

### Modes of the Invention

Hereinafter, the present invention will be described in detail with reference to examples to help in understanding the present invention. However, the following examples are merely provided to illustrate the content of the present invention, and the scope of the present invention is not limited to the following examples. The examples of the present invention are provided to more completely explain the present invention to those of ordinary skill in the art.

### <Example 1> Production and verification of human induced pluripotent stem cell-derived skin organoid

Culture of human induced pluripotent stem cells: Human induced pluripotent stem cell lines (iPSCs; CMC3, CMC11) were cultured on a vitronectin (ThermoFisher)-coated culture dish in a Y-27632 (10 µM)-supplemented Essential 8 (Gibo) medium. The medium was exchanged daily, and subculture was performed using ReLeSR (StemCell Technologies) every 4 days.

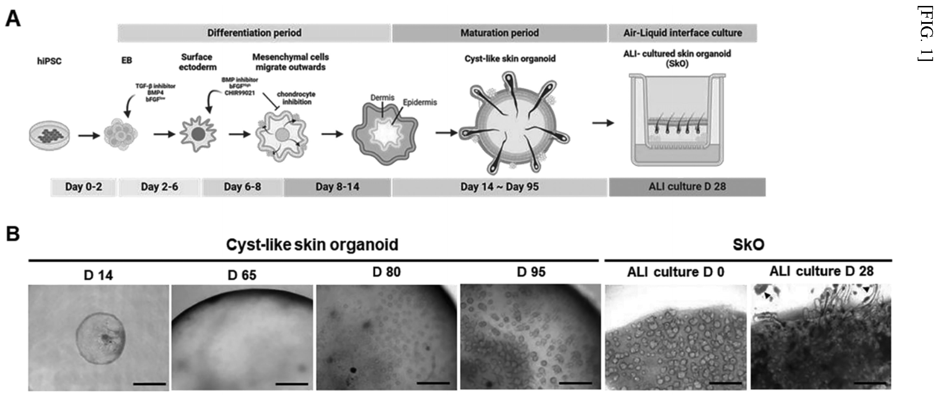

Skin organoid differentiation method using human induced pluripotent stem cell line: iPSC colonies were separated into single cells using Acuutase (Gibco). To form an embryoid body, 1500 single cells were dispensed into each well of a U-bottom low-attachment 96-well plate (SPL). The embryoid body medium was StemFlex (Gibco) containing Y-27632 (20 µM). The cells were cultured until the embryoid body grew to a size of 250 to 300 µm. To induce non-neural ectoderm differentiation, the formed embryoid was transferred to a medium Essential 6 (Gibco) containing 2% Matrigel (Corning), 10 µM SB431542 (Tocris), 4 ng/mL FGF2 (PeproTech) and 2.5 to 15 ng/mL of BMP4 (PeproTech). To induce cranial neural crest cells from day 3 or day 4, 200 nM LDN193189 (Torcris), 50 ng/mL of FGF2, and 3 µM CHIR-99021 were added to the medium. In addition, on day 12, the organoid medium was exchanged with a skin organoid maturation medium. The medium was exchanged every 3 days. On day 20, the organoids were transferred to a 6-well plate (low-attachment 6-well plate; SPL) containing 3 mL of skin organoid maturation medium, and skin organoids were cultured in an orbital shaker (65 rpm; ThermoFisher). The medium was exchanged every 3 days. The skin organoid maturation medium includes 1X GlutaMAX (Gibco), 0.5X B-27 minus vitamin A (Gibco), 0.5X N2 (Gibco), 0.1mM 2-mercaptoethanol (Gibco), and 100 µg/ml Primocin (InvivoGen) in a 1:1 Advanced DMEM/F12 (Gibco) and Neurobasal (Gibco) medium. After culturing the skin organoids for about 40 to 100 days, the organoids were cut into 4 to 12 uniformly-sized pieces using Dumount #3 forceps (Fine Science Tools) and spring scissors (Fine Science Tools), and spread on a collagen (Corning)-coated Transwell culture insert (pore size: 0.4 µm, 12-well plate (Corning)) such that the dermis faced the collagen side and the epidermis was exposed to the air, and cultured. The ALI-skin organoids were further cultured with a skin maturation medium in an incubator under a 100% humidity condition for 3 to 4 weeks in an air-liquid interface (air-liquid interface) state, and then cultured under a dry condition (0% humidity condition) for 2 to 6 days for maturation of the stratified epithermal layer (FIG. 1A and FIG. 1B).

Verification of skin organoid characteristics: The skin structure shown in the fabricated skin organoid was compared with the skin structure of a human adult. H&E staining and immunofluorescence staining were performed to confirm the stratum basale (KRT5) and the squamous epithelium (KRT10) of the epidermis, and the expression of skin barrier markers such as filaggrin (stratum corneum, squamous epithelium) and loricrin (cornified layer) were confirmed, indicating the similarity to the epidermis of actual skin.

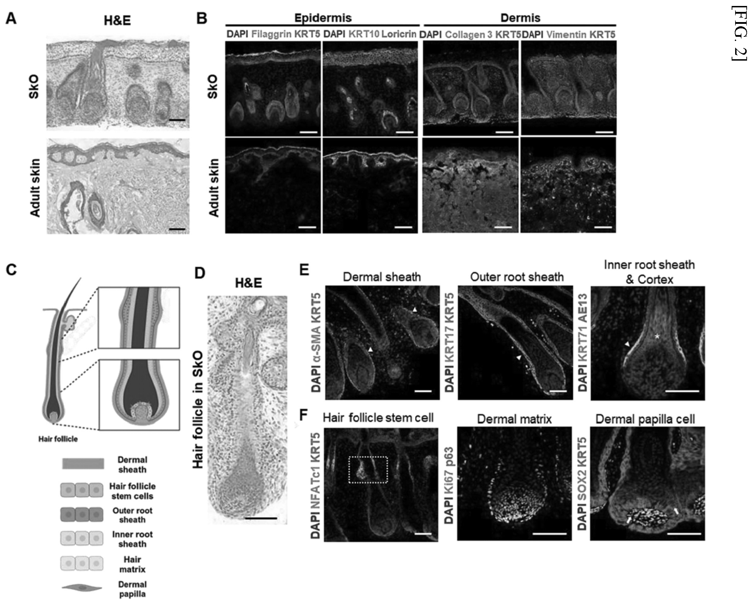

In addition, the expression of both collagen 3, which is a major structural protein mainly expressed in the dermis, and vimentin, which is one of the intermediate filaments of the skin, was confirmed, verifying that the skin organoid is similar to actual human skin (FIG. 2A and FIG. 2B).

As a result of performing H&E and immunofluorescence staining to verify the structural characteristics of a hair follicle in the skin organoid, the microstructures of a hair follicle, such as the dermal sheath (α-SMA), the outer root sheath (KRT17), the inner root sheath (KRT71), and the follicular cortex (AE13), were confirmed, and all of the dermal papilla cells (SOX2), the hair matrix cells (Ki67, P63), and the bulge area (NFATc1), which participate in the growth and function of hair follicles, were confirmed (FIG. 2C to FIG. 2F).

### <Example 2> Establishment of skin damage model by solar light-mimicking UV irradiation

There are two types of sUV, which reach the skin, UV-A and UV-B. UV-A can penetrate from the epidermis to the deepest layer of the dermis, while UV-B can only penetrate the epidermis and the upper part of the dermis. sUV exposure can destroy extracellular matrix (ECM) components and key proteins, causing signs of aging such as sunburn and pigmentation. To reproduce sUV-induced skin damage *in vitro* by mimicking sUV exposure, SkOs were irradiated a total of three times with a combination of UV-A and UV-B (94.5% UV-A and 5.5% UV-B) every two days.

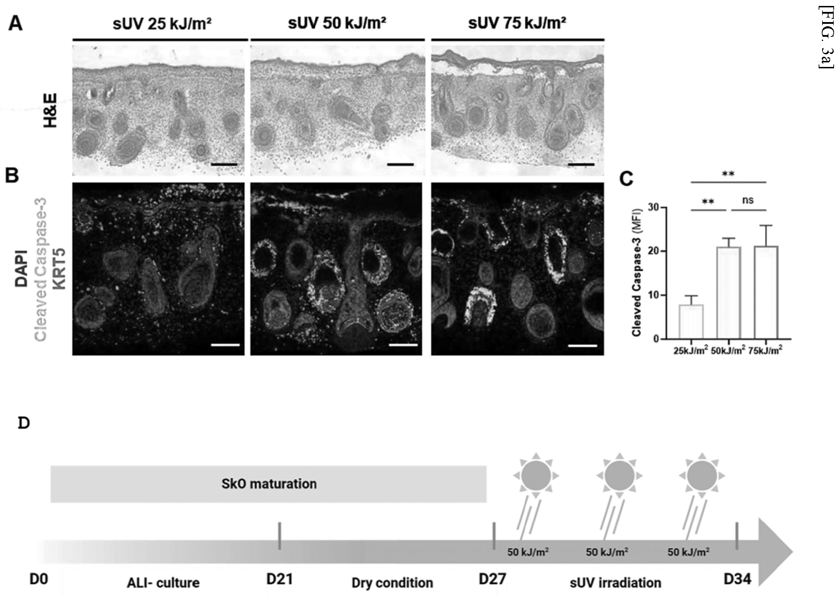

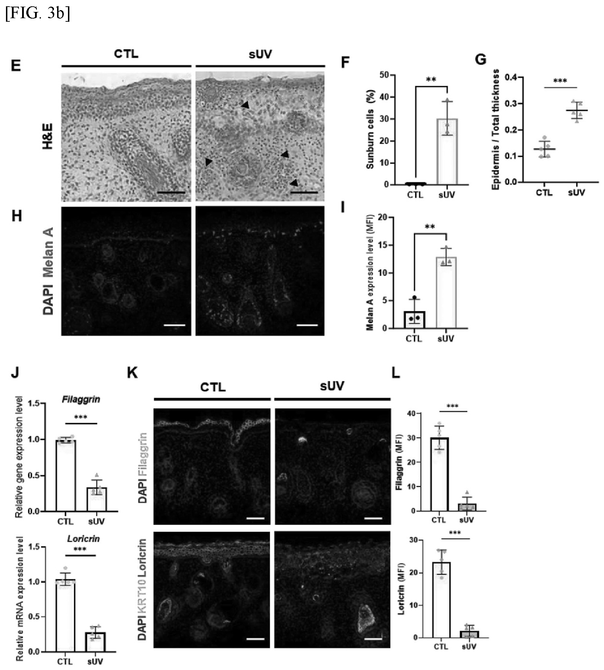

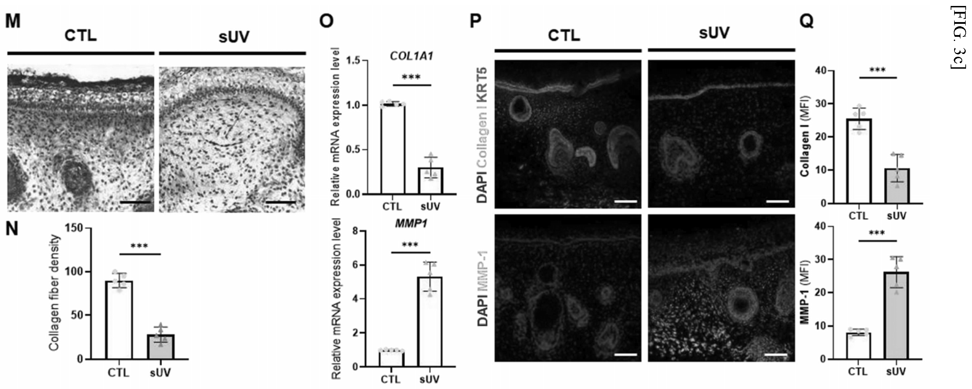

First, the effect of various sUV doses on SkOs was investigated. As a result, the entire epidermis of SkOs was peeled off by a high dose (75 kJ/m²) of sUV, indicating that the structural integrity of the skin was significantly damaged (A of FIG. 3A). As a result of immunofluorescence staining, the extent of dermal damage induced by 50 kJ/m² sUV was similar to the damage caused by 75 kJ/m² sUV, whereas 25 kJ/m² sUV did not cause severe damage to SkOs (B and C of FIG. 3A). Therefore, 50 kJ/m² sUV was determined to be the optimal dose to induce photodamage in SkOs (D of FIG. 3A).

After sUV exposure, the number of sunburned cells and epidermal thickness, characterized by fragmentation or condensation of the cell nucleus, were significantly increased in both the epidermis and dermis of SkOs (E to G of FIG. 3B). In addition, the number of melanocytes in the epidermis and hair follicles increased compared to the control SkOs, indicating skin pigmentation (H and I of FIG. 3B). Particularly, sUV also affected the outer epidermal layer of SkOs, which acts as a protective barrier for the skin. The mRNA expression of filaggrin and loricrin, which are skin barrierrelated genes, was significantly reduced in SkOs exposed to sUV, suggesting impaired skin barrier function after irradiation (J of FIG. 3B). Consistently, proteins forming the epidermal barrier, such as filaggrin, loricrin and CK10, were significantly downregulated in the epidermis of SkOs exposed to sUV, compared to the control SkOs (K and L of FIG. 3B).

Furthermore, the sUV effect extended to the deep dermal layer of SkOs. The density of collagen fibers was significantly reduced in the dermis of SkOs exposed to sUV (M and N of FIG. 3C). qPCR analysis showed a significant decrease in COL1A1 expression and an increase in matrix metalloproteinase-1 (MMP-1), which is an enzyme responsible for direct degradation of ECM fibers (O of FIG. 3C). Particularly, immunofluorescence staining showed that type I collagen expression was significantly reduced and MMP-1 expression was increased in the dermis of the SkOs exposed to sUV (P and Q of FIG. 3C). Based on the above results, sUV reaching the epidermis and the dermis caused overall skin damage in SkOs. Therefore, the present inventors successfully established in vitro conditions to reproduce sUV-induced photodamage in SkOs.

### <Example 3> Evaluation of hair follicle damage by solar light-mimicking UV irradiation

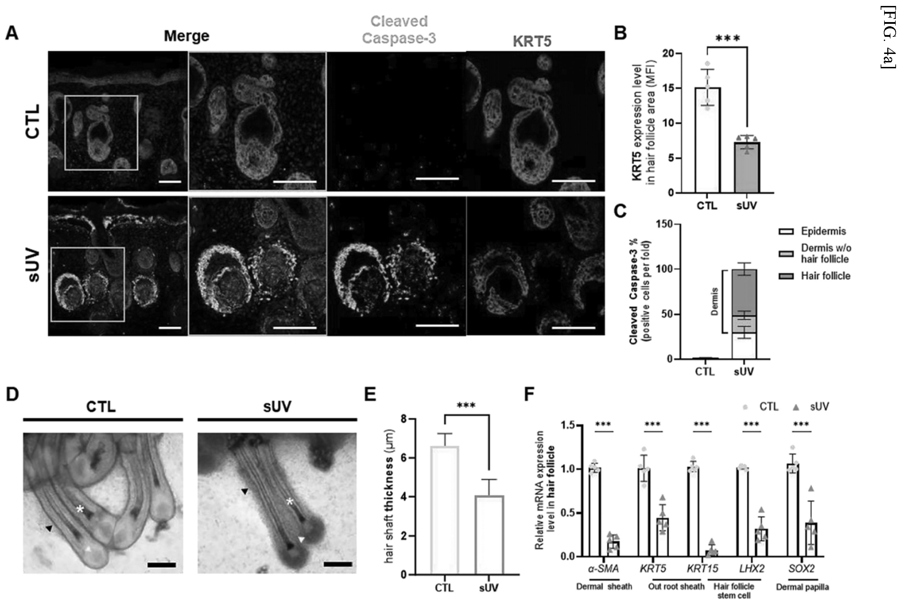

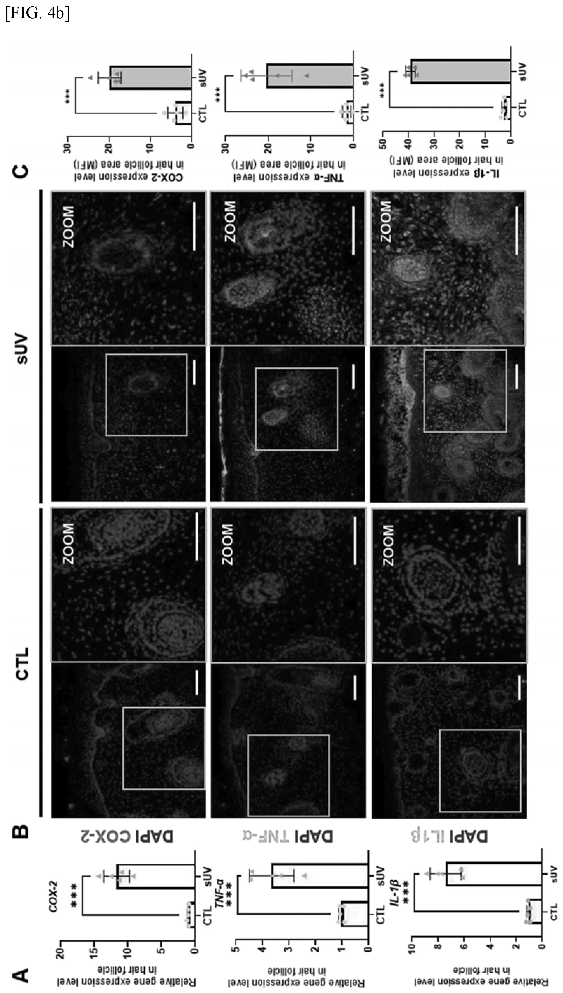

The effects of sUV exposure on skin appendages in the dermis of SkOs were investigated. As a result, it was confirmed that the expression of a KRT5 marker constituting the outer layer of a hair follicle in SkOs was reduced. Furthermore, apoptosis was significantly increased in SkOs, especially in cells that co-localize with KRT5-expressing cells in hair follicles (A, B, and C of FIG. 4A). It was visually observed that the dermal sheath (DS) and outer root sheath (ORS) of hair follicles were significantly destroyed, and the hair shaft was relatively thinner in the sUV group, indicating that the hair follicle had transitioned from anagen morphology to telogenlike morphology (D and E of FIG. 4A). Accordingly, to thoroughly compare the extent of hair follicle damage, hair follicles were isolated from each skin organoid and subjected to qRT-PCR analysis. The gene expression of a DS marker (a-SMA), ORS markers (KRT5, KRT15), hair follicle stem cell markers (KRT15, LHX2), and a DP marker (SOX2) was significantly reduced in the sUV group, indicating that sUV can penetrate the dermis and adversely affect hair follicles (F of FIG. 4A).

It is known that sUV irradiation causes the secretion of pro-inflammatory cytokines in skin tissue, leading to skin inflammation and subsequent skin damage. Accordingly, it was investigated whether sUV exposure can also affect hair follicles and induce an inflammatory response in the dermis of SkOs. First, the gene expression of pro-inflammatory cytokines, such as COX-2, TNF-α, and IL-1β, significantly increased in hair follicles in sUV-exposed SkOs (G of FIG. 4B).

Next, immunofluorescence analysis was performed to identify local inflammatory responses in SkOs. As a result, increased production of pro-inflammatory cytokines was expressed throughout the skin layer, but was more pronounced within the hair follicles of sUV-irradiated SkOs (H and I of FIG. 4B). These results suggest that sUV irradiation induces structural damage and an inflammatory response in the hair follicles of SkOs.

Overall, the present invention demonstrates that sUV exposure can sensitize and destroy not only skin tissue but also hair follicles responsible for hair growth.

### <Example 4> Derivation and evaluation of therapeutic candidate material using skin organoid model damaged by UV rays

The possibility of applying sUV-damaged SkOs as a photodamage mechanism study model for potential drugs was explored. Currently, exosomes derived from hUCB-MSCs are considered to be therapeutic drug candidates for various skin problems due to the ability to promote skin regeneration and reduce skin inflammation. In this context, the effect of UCB-Exos on photodamage was investigated using the sUV-exposed SkO models.

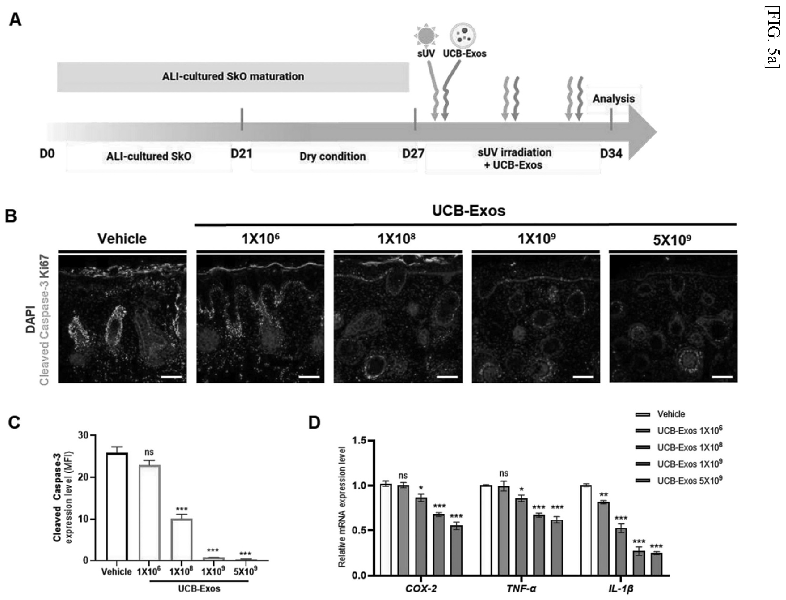

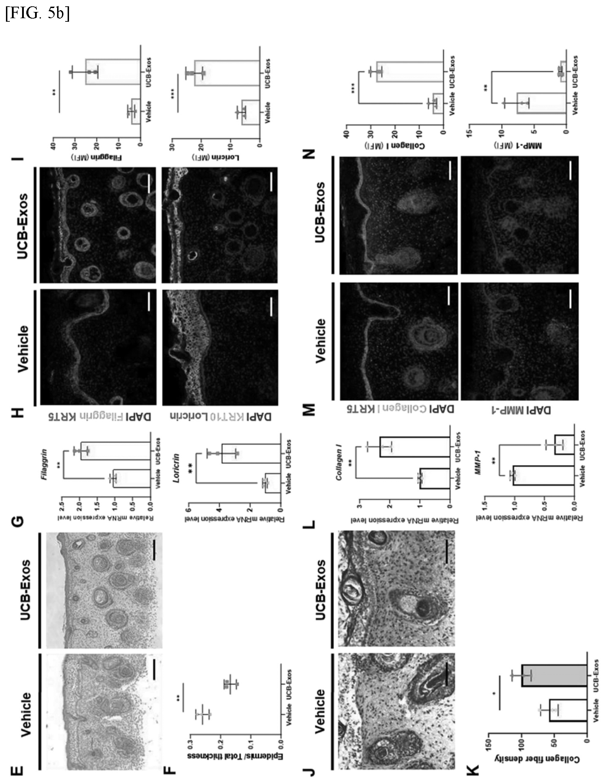

To determine the optimal concentration of UCB-Exos for alleviating SkO damage by sUV, SkOs were treated with different concentrations of UCB-Exos two hours after sUV exposure, and this procedure was repeated three times (A of FIG. 5A). As a result, SkO damage was effectively reduced in a dose-dependent manner by UCB-Exos treatment.

Particularly, when treated with 1 × 10⁸ particles or more of UCB-Exos, sUV exposure-induced apoptosis in hair follicles was effectively alleviated in the dermis of SkOs (B and C of FIG. 5A). However, a more significant effect on alleviating the inflammatory response was observed at a UCB-Exos concentration of 1 × 10⁹ particles (D of FIG. 5A). Based on the above results, it was confirmed that at least 1 × 10⁹ particles of UCB-Exos are required to fully exert the beneficial effects of inhibiting apoptosis, restoring skin barrier function, and regulating the secretion of inflammatory cytokines.

UCB-Exos (1 × 10⁹) treatment effectively reduces the epidermal thickness (E and F of FIG. 5B), and alleviates the disruption of the skin barrier proteins (filaggrin and loricrin), indicating that epidermal damage caused by sUV exposure was improved in SkOs (G, H, and I of FIG. 5B).

The density of collagen fibers reduced after sUV exposure was restored (J and K of FIG. 5B). Consistently, MMP-1 expression decreased and COL1A1 expression increased in UCB-Exos-treated SkOs (L of FIG. 5B). As a result of immunofluorescence analysis, it was confirmed that the dermis of the UCB-Exos-treated SkOs maintained higher levels of collagen fibers and MMP-1 expression was reduced, suggesting that collagen degradation was inhibited in sUV-damaged SkOs (M and N of FIG. 5B).

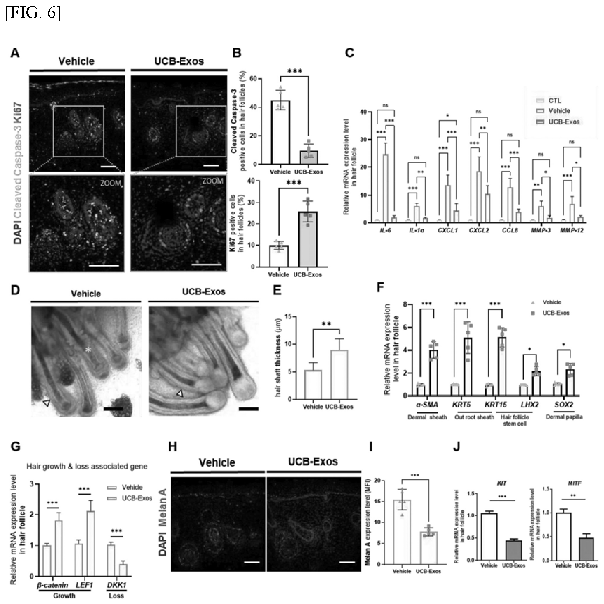

The effect of UCB-Exos on hair follicles in SkOs was investigated. In the hair follicles, apoptosis (cleaved caspase-3⁺) was reduced, and the number of regenerating cells (Ki67⁺) increased (A and B of FIG. 6). In addition, keratinocytes and fibroblasts in the hair follicles aged by sUV are known to secrete MMPs and inflammatory cytokines known as a senescence-associated secretory phenotype (SASP). SASP-related genes were significantly downregulated in hair follicles of the UCB-Exos group, indicating a younger and healthier condition (C of FIG. 6). These results suggest that UCB-Exos effectively mitigates sUV-induced hair follicle cell death and aging, and promotes hair follicle regeneration.

sUV-induced hair follicle damage, for example, shrunken hair bulbs and the disruption of the outer walls such as the DS and ORS, was clearly recovered from in the UCB-Exos-treated group. In addition, abnormal hair shaft entry into anaphase was restored after UCB-Exos treatment (D and E of FIG. 6). Consistently, gene expression related to the structural components of hair follicles was upregulated in the UCB-Exos group (F of FIG. 6). Furthermore, hair growth-related genes (β-catenin and LEF1) were upregulated, and a hair loss-related gene (DKK1) was downregulated, suggesting that UCB-Exos restored the hair formation ability of SkOs (G of FIG. 6).

Melanocytes were also affected by UCB-Exos treatment. In both the epidermis and hair follicles, the number of melanocytes decreased (H and I of FIG. 6), and the expression of genes (KIT and MITF) associated with melanocyte development and pigmentation was also reduced (J of FIG. 6).

In summary, the present invention demonstrates the therapeutic potential of UCB-Exos to improve skin damage and promote hair follicle regeneration in sUV-exposed SkOs.

### <Example 5> Confirmation of IκB/NF-κB-mediated damage and treatment mechanism using UV-damaged skin organoid model

Several studies have suggested that the exposure to sUV radiation activates the inflammatory chain reaction in the skin, leading to apoptosis. NF-κB is known to play an important role in regulating the expression of immune mediators. In an inactive state, NF-κB binds to an inhibitory protein called IκBα to form an NF-κB-IκB complex in the cytoplasm. When activated by external stimuli such as inflammatory cytokines or cellular stress, the IκBα protein is phosphorylated and subsequently degraded, releasing NF-κB from the NF-κB-IκB complex. The released NF-κB translocates to the cell nucleus to promote the transcription of genes involved in inflammation and induce cytokine production. Considering the above, the present inventors investigated whether IκB-dependent NF-κB activation mediates the inflammatory response in hair follicles.

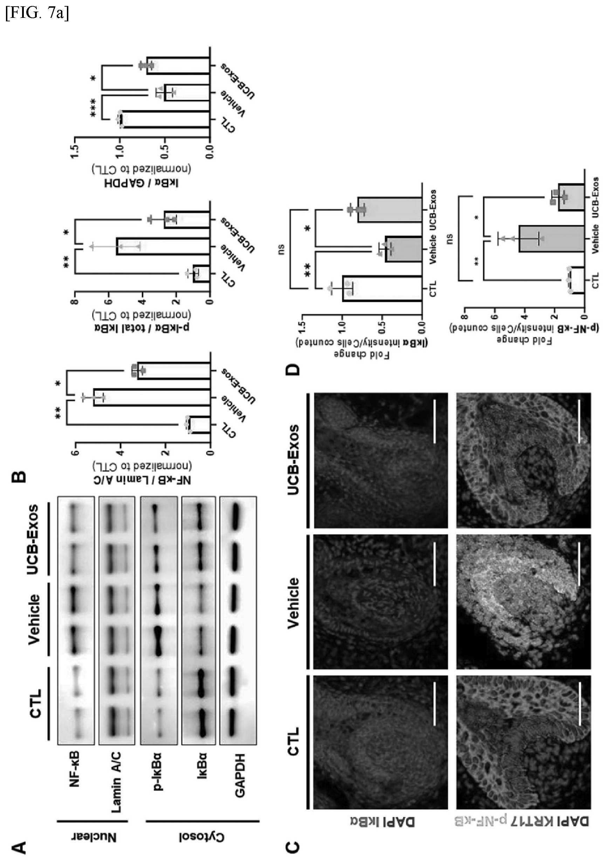

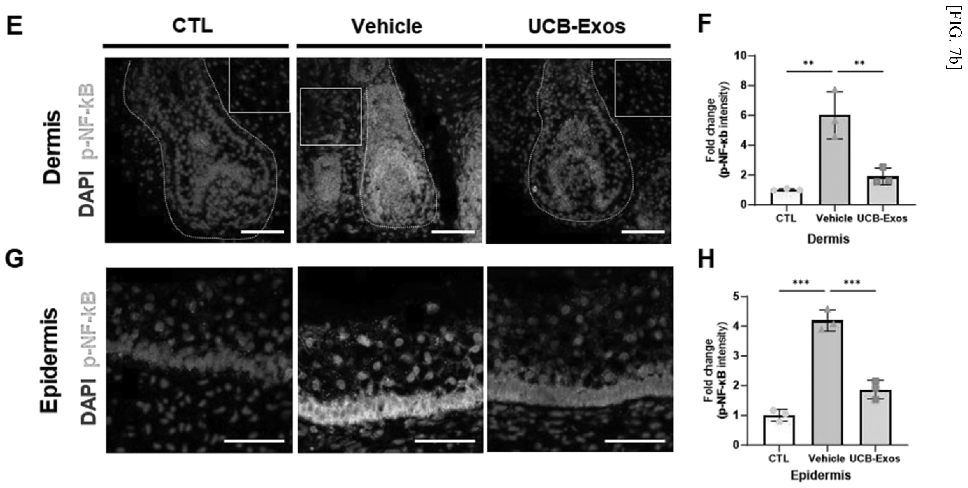

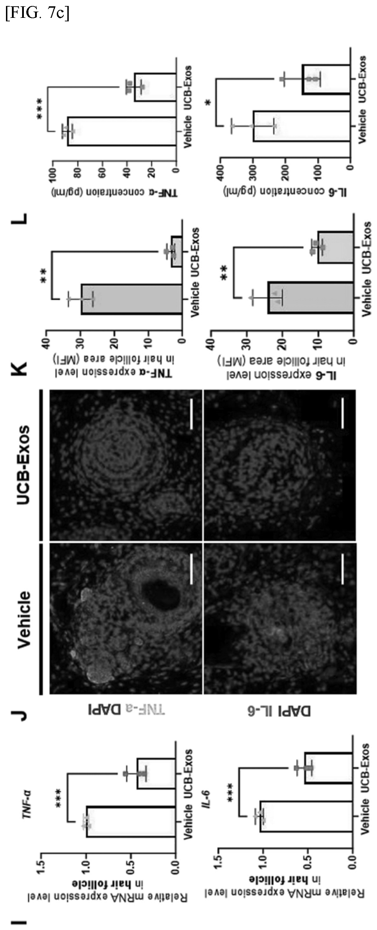

The present inventors found that sUV induced IκBα phosphorylation in the cytoplasmic fraction of SkOs and subsequent IκBα degradation. This caused the disruption of the NF-κB-IκB complex which activated NF-κB and caused it to translocate to the nucleus, as evidenced by increased NF-κB expression in the nuclear compartment of SkOs exposed to sUV. After treating the sUV-exposed SkOs with UCB-Exos, it was observed that both IκBα phosphorylation and degradation decrease, and nuclear translocation of NF-κB was inhibited (A and B of FIG. 7A). Immunofluorescence staining also revealed the localized expression of IkBα and activated NF-κB, i. e, phosphorylated NF-κB (p-NF-κB) in cells constituting the hair follicle structure. Generally, hair follicles are composed of keratinocytes and fibroblasts, both of which play an important role in skin inflammatory responses. Particularly, in the sUV-treated group, it was observed that IkBα expressed throughout the hair follicles including keratinocytes of the ORS was significantly inhibited. Accordingly, sUV exposure significantly increased p-NF-κB expression in the ORS and the hair follicle matrix (C and D of FIG. 7A). In these structures, the nuclear translocation of p-NF-κB also increased in sUV-induced SkOs, indicating NF-κB activation after sUV exposure. Notably, the NF-κB-mediated inflammatory response occurred mainly in hair follicles rather than in dermal fibroblasts (E and F of FIG. 7B). Meanwhile, the epidermis of SkOs, which is the uppermost layer exposed to sUV, was also inflamed, which may extend to the dermis and indirectly damage hair follicles (G and H of FIG. 7B). These expression patterns of IkBα and NF-κB were effectively reversed by UCB-Exos treatment (E to H of FIG. 7B).

In addition, the mRNA expression of inflammatory genes TNF-α and IL-6 was significantly reduced in isolated hair follicles after UCB-Exos treatment (I of FIG. 7C). High TNF-α and IL-6 protein levels exhibited throughout the hair bulbs in a vehicle control were significantly reduced in the matrix region after UCB-Exos treatment (J and K of FIG. 7C). This was consistently reflected in the secretion of TNF-α and IL-6 of SkOs confirmed through ELISA (L of FIG. 7C). Overall, these results suggest that UCB-Exos alleviated the inflammatory response in fibroblasts and keratinocytes in hair follicles by inhibiting IκB degradation and NF-κB activation induced by sUV exposure.

### <Example 6> Mediation of therapeutic efficacy of exosomes against hair follicle photodamage by inhibition of NF-κB signaling

To evaluate the direct effect of UCB-Exos alleviating sUV-induced damage by NF-κB activation, the present inventors treated a sUV-damaged organoid with the NF-κB inhibitor, BAY11-7082. BAY11-7082 is known to inhibit the translocation of NF-κB to the nucleus by inhibiting the degradation of IκBα unit, thereby preventing further activation of NF-κB signaling.

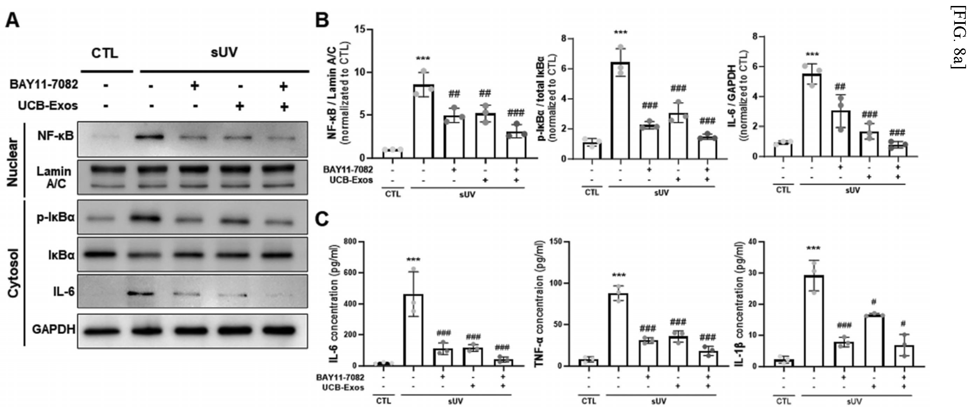

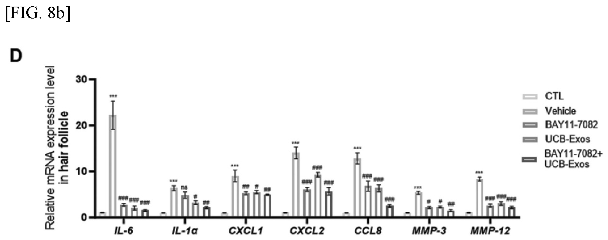

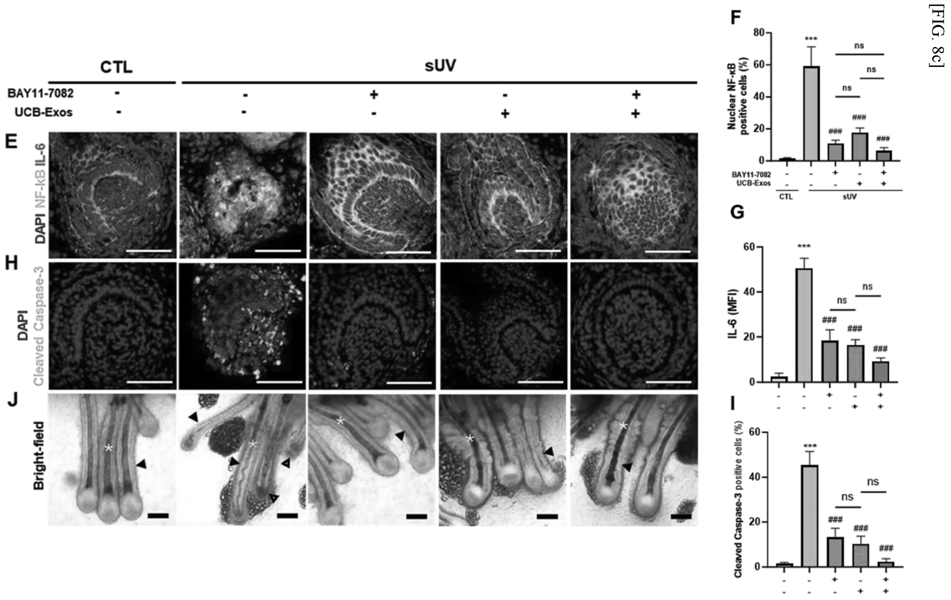

Western blotting revealed that, after BAY11-7082 treatment, sUV-damaged SkOs had decreased phosphorylated IκBα, leading to inhibition of IκBα degradation and thus reduced nuclear translocation of NF-κB. Similarly, sUV-exposed SkOs supplemented with UCB-Exos significantly reduced IκBα phosphorylation, resulting in reduced IκBα degradation and NF-κB activation. Particularly, co-treatment with BAY11-7082 and UCB-Exos synergistically reduced NF-κB and phosphorylated IκBα levels. As expected, it was confirmed that IL-6 expression was positively correlated with the expression level of NF-κB (A and B of FIG. 8A).

Furthermore, the secretion of inflammatory cytokines, such as IL-6, TNF-α, and IL-1β, increased by sUV exposure, was evaluated by ELISA. There was no significant difference in IL-6 and TNF-α secretion between BAY11-7082-treated SkOs and UCB-Exos-treated SkOs, but TNF-α and IL-1β levels were significantly reduced in the co-treated group, indicating a synergistic effect of BAY11-7082 and UCB-Exos (C of FIG. 8A).

In the context of inflammatory responses, NF-κB signaling is known to be a key regulator of the SASP component, which delays tissue regeneration through accumulation of senescent cells, abnormalities in matrix remodeling, and chronic inflammation.

It was examined whether UCB-Exos treatment, which inhibits NF-κB activation, can regulate SASP in hair follicles. As a result, it was shown that the mRNA expression of ECM components associated with inflammatory cytokines and SASP is dramatically reduced after treatment with UCB-Exos or BAY11-7082 (D of FIG. 8B). This result suggests that the inhibition of NF-κB activation by UCB-Exos has a potential to mitigate the detrimental effects of SASP, thereby having a more favorable environment for tissue regeneration.

Immunofluorescence staining revealed a significant decrease in both NF-κB translocation to the nuclei and IL-6 in the cytoplasm in hair follicles after BAY11-7082 or UCB-Exos treatment (E, F, and G of FIG. 8C). As a result, it was confirmed that apoptosis was reduced in these hair follicles after BAY11-7082 or UCB-Exos treatment, and after co-treatment, the death rate was the lowest (H and I of FIG. 8C). Ultimately, NF-κB inhibition restored the structural integrity of hair follicles including the DS, ORS, and hair bulbs, and protected hair follicles from transitioning to the anagen phase (J of FIG. 8C). In summary, the present invention suggests that UCB-Exos effectively restored sUV-damaged hair follicles through NF-κB inhibition.

### <Example 7> Confirmation of hair follicle growth promoting effect by NF-κB signaling inhibitor in normal hair follicle organoid

To evaluate the effect of NF-κB signaling inhibition in normal hair follicles, hair follicle growth was observed after treating a skin organoid in which hair follicles were formed with the NF-κB inhibitor, BAY11-7082. The hair follicle growth was compared after treatment with each of minoxidil, which is known to promote hair follicle growth, and dihydrotestosterone (DHT), which is an endogenous hormone causing hair loss.

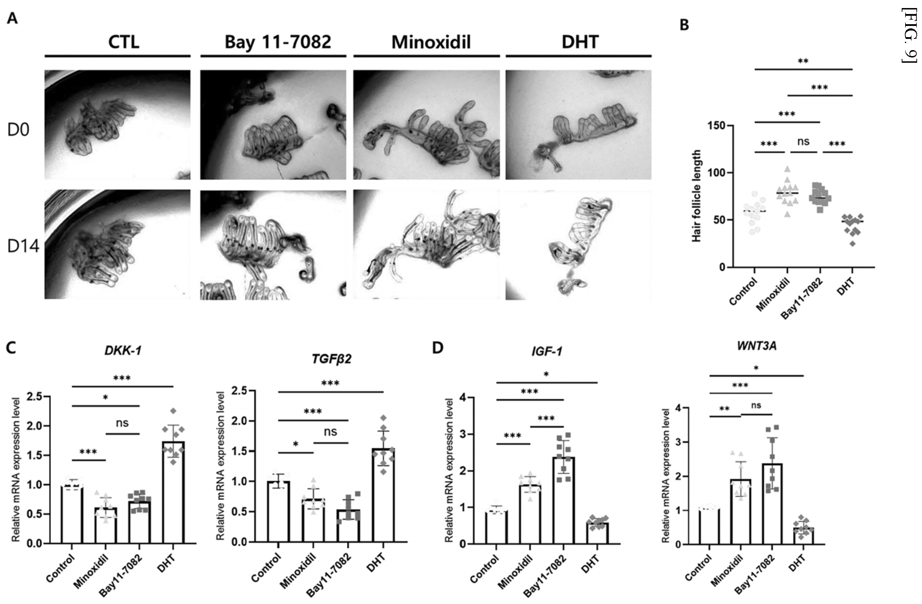

As a result of observing the morphology of hair follicles 14 days after drug treatment, a hair follicle length increased approximately 1.4-fold in the BAY11-7082-treated group, and increased approximately 1.3-fold in the minoxidil-treated group, compared to the control. Conversely, it was confirmed that the hair follicle length decreased approximately 0.7-fold in the DHT-treated group compared to the control. This showed that the NF-κB inhibitor BAY11-7082 promoted hair follicle growth similarly to minoxidil (FIG. 9A and FIG. 9B).

Additionally, the expression of genes (IGF-1, WNT3A) associated with hair growth promotion was upregulated compared to the control, while hair loss-associated genes (DKK1, TGFb-2) were downregulated. This suggests that the NF-κB inhibitor BAY11-7082 has the effect of promoting hair follicle growth (FIG. 9C).

The present invention demonstrated that BAY11-7082 can promote hair follicle growth through NF-κB signaling inhibition even in normal follicles, and confirmed its potential as a novel therapeutic agent for promoting hair growth.

In conclusion, based on the above evidence, the present invention has revealed that skin damage occurring due to an actual external environmental factor can be mimicked in an *in vitro* model through the mechanisms occurring in actual skin by utilizing a human induced pluripotent stem cell-derived skin organoid. In addition, as it was verified that several phenotypes of the disease were alleviated when the efficacy of candidate materials that could possibly be used as a therapeutic agent in UV-induced skin damage models was evaluated, it was confirmed that the above skin organoid can be used as a drug screening platform. The present invention will contribute to the improvement of patients' lives by being applied to research on skin damage and aging caused by UV rays, and it is also expected that the present invention can be widely used in drug and toxicity evaluations, preclinical research, regeneration, tissue engineering research, etc.

It should be understood by those of ordinary skill in the art that the above description of the present invention is exemplary, and the embodiments disclosed herein can be easily modified into other specific forms without changing the technical spirit or essential features of the present invention. Therefore, it should be interpreted that the embodiments described above are exemplary in all aspects and not limiting. The scope of the present invention is indicated by the following claims, and all changes or modifications derived from the meaning and scope of the claims and their equivalent concepts should be interpreted as being included in the scope of the present invention.

### Industrial Applicability

The present invention relates to a skin damage organoid model and a drug screening method using the same, and therefore, skin damaged by an external environment (UV rays) was modeled by utilizing a human induced pluripotent stem cell-derived skin organoid. By utilizing organoid technology that mimics the actual human development process, a skin organoid that contains cells constituting skin, cells constituting hair follicles, and all microstructures without modification was produced and irradiated with UV rays to mimic an external stimulus in an actual external environment, establishing a technology to model damaged skin in vitro. Therefore, through this technology, a mechanism causing damage and aging caused by UV rays may also be confirmed in vitro, and a platform that can derive a candidate material capable of alleviating this was developed. The platform established through the present invention can be applied as a platform that can evaluate and derive the mechanism of disease occurrence and the efficacy of various drug candidates in the future.

## Claims

1. A method of preparing a skin damage organoid, comprising:
(1) culturing a pluripotent stem cell-derived organoid in the presence of a Wnt agonist;
(2) culturing the cultured product obtained in (1) in a skin organoid maturation medium;
(3) preparing a skin organoid by cutting the cultured product obtained in (2) and culturing it at an air-liquid interface; and
(4) irradiating the skin organoid with solar light-mimicking UV rays,
wherein the Wnt agonist is added at the beginning of induction of differentiation of non-neural ectoderm into cranial neural crest cells (CNCCs).

2. The method of claim 1, wherein the skin damage organoid is a hair follicle damage organoid in which hair follicles among appendages of the skin are damaged.

3. The method of claim 1, wherein the Wnt agonist is selected from the group consisting of CHIR-99021, WNT3A, WNT5A, and RSPO1.

4. The method of claim 1, wherein the Wnt agonist is added on day 5 to day 7 of the culture of pluripotent stem cells.

5. The method of claim 1, wherein, in (3), the cultured product obtained in (2) may cut into four equal-sized pieces and cultured at the air-liquid interface on a collagen-coated Transwell culture insert such that the dermis faces the collagen side and the epidermis is exposed to the air.

6. The method of claim 1, wherein the skin damage organoid is prepared by damaging skin through NF-kB activation.

7. A skin damage organoid prepared by the method of claim 1.

8. A method of screening a skin damage treatment agent, preventive agent or improvement agent, comprising:
treating the skin damage organoid of claim 7 with a candidate material for a skin damage treatment agent, preventive agent or improvement agent.

9. The method of claim 2, wherein the hair follicle damage organoid is prepared by damaging hair follicles through NF-kB activation.

10. The method of claim 2, wherein, in the hair follicle damage organoid, the expression of one or more genes selected from the group consisting of a-SMA, which is a dermal sheath marker, KRT5 or KRT15, which is an outer root sheath marker, LHX2, which is a hair follicle stem cell marker, and SOX2, which is a dermal papilla marker, is reduced.

11. The method of claim 2, wherein the hair follicle damage organoid has an increase in gene expression of a pro-inflammatory cytokine, such as COX-2, TNF-α, or IL-1β.

12. A hair follicle damage organoid prepared by the method of claim 2.

13. A method of screening a hair growth promoter, a hair loss preventive agent, a hair loss relief agent, or a hair loss treatment agent, comprising:
treating the hair follicle damage organoid of claim 12 with a candidate material of a hair growth promoter, a hair loss preventive agent, a hair loss relief agent or a hair loss treatment agent.

14. The method of claim 13, wherein the candidate material inhibits NF-kB activated in the hair follicle damage organoid.

15. A biomarker composition for detecting hair follicle damage, comprising NF-kB as an active ingredient.

16. A kit for detecting hair follicle damage, comprising an agent for detecting NF-kB.

17. The kit of claim 16, wherein the agent is selected from one or more selected from the group consisting of a primer, a probe, an antisense oligonucleotide, an aptamer, and an antibody, which are specific for NF-kB.

18. A method of providing information required for hair follicle damage detection, comprising:
(1) measuring an expression level of NF-kB from an isolated sample;
(2) comparing the expression level of NF-kB with that of a control sample; and
(3) determining that hair follicles are damaged when the expression level of NF-kB is higher than that of the control sample.

19. A pharmaceutical composition for a hair growth promoter, a hair loss preventive agent, a hair loss relief agent, or a hair loss treatment agent, comprising an NF-kB activation or expression inhibitor as an active ingredient.

20. The pharmaceutical composition of claim 19, wherein the NF-kB activation inhibitor is any one selected from the group consisting of a small molecule compound, a peptide, a peptide mimetic, an aptamer, an antibody, and a natural substance, which specifically bind to the NF-kB protein.

21. The pharmaceutical composition of claim 19, wherein the NF-kB expression inhibitor is any one selected from the group consisting of an antisense nucleotide, small interfering RNA (siRNA), and short hairpin RNA (shRNA) which complementarily bind to mRNA of the NF-kB gene.

22. A pharmaceutical composition for a hair removal agent, comprising an NF-kB activation or expression promoter as an active ingredient.

23. The pharmaceutical composition of claim 22, wherein the NF-kB activation promoter is any one selected from the group consisting of a small molecule compound, a peptide, a peptide mimetic, an aptamer, an antibody, and a natural substance, which specifically bind to the NF-kB protein.

24. The pharmaceutical composition of claim 22, wherein the NF-kB expression promoter is any one selected from the group consisting of adeno-associated virus (AAV), adenovirus, lentivirus, and retrovirus, which include an NF-kB coding gene increasing the expression of mRNA of the NF-kB gene.

25. A cosmetic composition for a hair removal agent, comprising an NF-kB activation or expression promoter as an active ingredient.

26. A method of promoting hair growth, preventing hair loss, reducing hair loss, or treating hair loss, comprising:
administering an effective amount of an NF-kB activation or expression inhibitor to a subject in need thereof.

27. A method of removing hair, comprising:
administering an effective amount of NF-kB activation or expression promoter to a subject in need thereof.
